# EUROPÄISCHE PATENTSCHRIFT

(11) **EP 0 533 969 B1**
(45) Veröffentlichungstag und Bekanntmachung des Hinweises auf die Patenterteilung: **20.07.1994**
(21) Anmeldenummer: 91116200.6
(22) Anmeldetag: 24.09.1991
(51) Int. Cl.: A61F 5/01, A43B 7/00

(54) **Orthopädische Redressionsschiene**
Orthopaedic correction splint
Stelle orthopédique de correction

(43) Veröffentlichungstag der Anmeldung: 31.03.1993
(73) Patentinhaber: IPOS GmbH & CO. KG., D-21337 Lüneburg (DE)
(72) Erfinder: Prahl, Jan, W-2127 Rullstorf (DE)
(74) Vertreter: Richter, Werdermann & Gerbaulet

(56) Entgegenhaltungen:
- EP-A- 0 027 762
- DE-A- 3 543 642
- DE-U- 9 107 481
- US-A- 2 967 360
- US-A- 4 434 792
- US-A- 4 538 599

## Beschreibung

Die Erfindung betrifft eine orthopädische Redressionsschiene zur Korrektur der Aduktion des Vor- oder Mittelfußes mit einer vorderen, den deformierten Fußteil zumindest seitlich umschließenden und einer hinteren Halbsohle, die gelenkig um eine durch einen Drehbolzen definierte Vertikale zur Halbsohlenebene schwenkbar miteinander verbunden sind und die eine Feder aufweisen, deren Federkraft in lateraler Richtung auf die vordere Halbsohle wirkt.

Die genannte Redressionsschiene, die auch als Sichelfußschiene bezeichnet wird, wird speziell für die Behandlung des angeborenen oder erworbenen Sichelfußes bei Kleinkindern eingesetzt. In einem frühen Behandlungsstadium gelingt es häufig, den Fuß allein mit den genannten Schienen in eine Normalstellung zu bringen. Bei schwereren Deformitäten oder späten Behandlungsstadien kann die Sichelfußschiene auch nach einer Fußoperation stützend verwendet werden. Soweit die Vorfußaduktion Teil einer Klumpfußdeformität ist, der u.a. mit einem Fersenhochstand einhergeht, muß die Redressionsschiene mit einer Wadenführung und einer Kniebeugefixierung verbunden werden, um den Klumpfuß erfolgversprechend behandeln zu können. Hinsichtlich der Gestaltung der Redressionsschiene besteht jedoch kein grundsätzlicher Unterschied. Die mit der genannten Redressionsschiene behandlungsfähige Fußdeformität kann in der Verformung des ersten Strahles, der nach medial abweicht, bestehen, während die übrigen vier Strahlen normal verlaufen oder in einer medialen Abweichung aller fünf Mittelfußknochen, dem typischen Sichelfuß.

Bei den nach dem Stand der Technik bekannten Redressionsschienen, wie sie u.a. in der EP-A-0 027 762 beschrieben werden, liegt der Drehbolzen auf der medialen Halbschalenseite, während auf der gegenüberliegenden Seite eine Zugfeder auf die beiden Halbsohlen derart einwirkt, daß die nach medial abweichenden Fußteile nach lateral gedrückt werden. Hierdurch ergeben sich folgende Nachteile. Wie bereits oben erwähnt, werden Sichelfußschienen häufig bei Kleinkindern eingesetzt, deren Fußknochen sich noch in der Wachstumsphase befinden. Durch die Drehbolzenanordnung an der medialen Seite wird diese Seite zwangsläufig stark komprimiert. Je nach Zugfederspannung wird somit der Großzeh stark gestaucht. Von medial nach lateral zunehmend wird eine starke Kompression der Fußwurzel bewirkt.

Es ist daher Aufgabe der vorliegenden Erfindung, die eingangs beschriebene Redressionsschiene dahingehend zu verbessern, daß bei umgestalteter Schiene ein verringerter Stauchungsdruck auf der lateralen Seite vorliegt.

Diese Aufgabe wird durch die im Anspruch 1 beschriebene orthopädische Redressionsschiene gelöst, deren erfindungsgemäße Kennzeichen darin besteht, daß der Drehbolzen an der Außenkante der beiden Halbsohlen, also lateral, angeordnet ist und daß die Feder eine Druckfeder ist, die auf der medialen Seite die Halbsohlen gegeneinander druckbelastet.

Durch die Verlagerung des Drehgelenkes auf die laterale Seite wird diese Seite erheblich vom Stauchungsdruck befreit, der bei den nach dem Stand der Technik bisher bekannten Konstruktionen vorlag. Die Fußwurzelknochen bleiben weitgehend entlastet. Gleichzeitig hat die Position des Drehpunktes auf der lateralen Seite eine fußaufrichtende Wirkung.

Weiterbildungen der Erfindung sind in den Unteransprüche 2 bis 8 beschrieben.

So liegt vorzugsweise der Drehbolzen in Höhe der Anlenkung des Kopfes des fünften Mittelfußknochens. Hierdurch wird eine optimale Druckaufbringung auf den Vorfuß gewährleistet.

Zwecks einfacher Bauweise besitzen die beiden Halbsohlen jeweils an ihrer Außenseite eine Verlängerung, wobei die Verlängerungen sich überlappen und von dem Drehbolzen durchdrungen sind. Weiterhin vorzugsweise befinden sich im Abstand von dem Drehbolzen zur Innenkante hin an jeder der beiden Halbsohlen vorbereitete Anlenkungspunkte für die Druckfeder, die insbesondere im Bereich der Innenkante der Halbsohle liegen. Es können jedoch auch mehr als zwei Anlenkungspunkte vorbereitet sein, etwa, um die Feder aus konstruktiven Gründen von der medialen Seite zur Mitte hin zu verlagern. Hierbei ist jedoch zu beachten, daß sich in entsprechender Weise der Hebelarm verkürzt.

Hinsichtlich der verwendeten Federart besteht grundsätzlich keine Beschränkung.In einer Weiterbildung der Erfindung wird jedoch eine Spiralfeder verwendet, deren endseitige Schenkel im wesentlichen parallel zur Außenkante der Halbsohle liegen.

Nach einer weiteren Ausgestaltung der Erfindung besitzen die Halbsohlen jeweils einen Winkel aus biegefestem Material, wobei die Winkel jeweils endseitig von dem Drehbolzen schwenkbar gehalten werden und deren freies Ende in Richtung der Innenkante und bis an diese heranreichend liegt. Diese Winkel, die z.B. aus Metall bestehen können, weisen jeweils vorbereitete Anlenkpunkte für die Druckfeder auf. Diese Ausgestaltung hat den Vorteil, daß insbesondere die schalenförmigen Fußbettflächen der Halbsohlen aus einem Material bestehen können, das sich unter Druck der Fußform anpaßt.

Nach einer weiteren Ausgestaltung der Erfindung ist vorgesehen, daß die Redressionsschiene als Schuh- oder Stiefelteil ausgebildet ist, wie dies prinzipiell in der EP-A-0 027 762 beschrieben wird.

Ausführungsbeispiele der Erfindung sind in den Zeichnungen dargestellt. Es zeigen

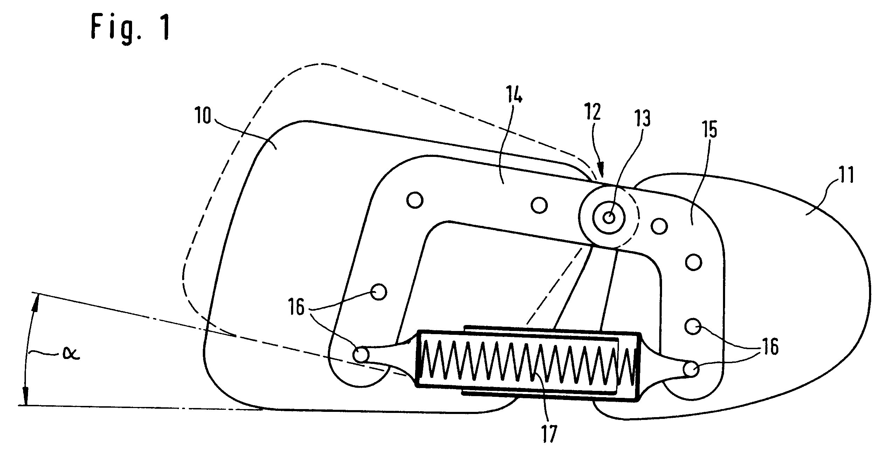

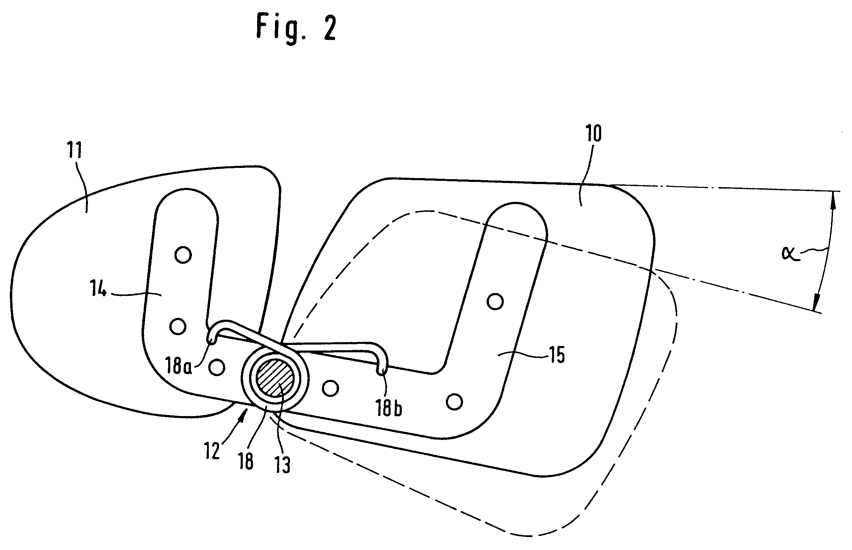

Fig. 1 und 2 jeweils prinzipielle Darstellungen von Redressionsschienen mit unterschiedlichen Druckfedern.

Die Redressionsschiene besteht aus einer vorderen Halbsohle 10 und einer hinteren Halbsohle 11, die über ein Drehgelenk 12 miteinander verbunden sind. Das Drehgelenk 12 besteht aus einem Drehbolzen 13, der jeweilige, sich überlappende Verlängerungen der vorderen und hinteren Halbsohle durchgreift. Diese Verlängerungen sind Teil jeweiliger Winkel 14 und 15 mit vorbereiteten Anlenkpunkten 16 für eine Druckfeder 17 (Fig.1) bzw. 18 (Fig.2).

Die Erfindung beinhaltet die konstruktive Ausgestaltung, daß sich das Drehgelenk an der Außenkante, der lateralen Seite, der Halbsohlen 10 und 11 befindet. In unbelastetem Zustand nehmen die Halbsohlen die mit durchgezogenen Linien dargestellte Stellung ein. Unter Belastung der Druckfedern 17 bzw. 18 schwenkt die vordere Halbsohle um einen Winkel α nach lateral aus, womit ein entsprechender Druck auf den Vorfuß ausgeübt wird.

Bei der Ausführungsform nach Fig.1 liegt die langgestreckte Druckfeder 17 im wesentlichen parallel und im Bereich der Innenkante der Halbsohlen 10 und 11, wobei die Druckfeder 17 jeweils endseitig an den freien Enden der Winkel 14 und 15 befestigt ist.

Bei der Ausführungsform nach Fig. 2 besteht die Druckfeder 18 aus einer Spiralfeder, deren freie federnden Enden 18a und 18b auf die Schenkel der Winkel 14 und 15 im lateralen Bereich wirken. Der übrige Teil der Druckfeder 18 ist medial um den Drehbolzen 13 angeordnet.

Wie sich aus einem Vergleich der mit durchgezogenen Linien dargestellten Kontur der vorderen Halbsohle 10 und der mit gestrichelten Linien dargestellten "geschwenkten" Kontur ergibt, wird bei Druckeinwirkung der Federn 17 und 18 die vordere Halbsohle "nach vorne" gezogen, wodurch der Fuß quasi in die Länge gezogen und eine Korrektur der Aduktionsstellung wesentlich begünstigt wird.

## Patentansprüche

1. Orthopädische Redressionsschiene zur Korrektur der Aduktion des Vor- oder Mittelfußes mit einer vorderen, den deformierten Fußteil zumindest seitlich umschließenden und einer hinteren Halbsohle (10,11), die gelenkig um eine durch einen Drehbolzen (13) definierte Vertikale zur Halbsohlenebene schwenkbar miteinander verbunden sind und die eine Feder (17,18) aufweisen, deren Federkraft in lateraler Richtung auf die vordere Halbsohle (10) wirkt, dadurch gekennzeichnet, daß der Drehbolzen (13) an der Außenkante der beiden Halbsohlen (10,11) angeordnet ist und daß die Feder eine Druckfeder (17,18) ist, durch die auf der medialen Seite die Halbsohlen (10,11) gegeneinander druckbelastet sind.

2. Orthopädische Redressionsschiene nach Anspruch 1 oder 2, dadurch gekennzeichnet, daß die beiden Halbsohlen (10,11) jeweils an ihrer Außenseite eine Verlängerung besitzen, wobei die Verlängerungen sich überlappen und von dem Drehbolzen (13) durchdrungen sind.

3. Orthopädische Redressionsschiene nach einem der Ansprüche 1 bis 2, dadurch gekennzeichnet, daß im Abstand von dem Drehbolzen (13) zur Innenkante hin jede der beiden Halbsohlen (10,11) mindestens einen vorbereiteten Anlenkpunkt (16) für die Druckfeder (17,18) aufweist.

4. Orthopädische Redressionsschiene nach Anspruch 3, dadurch gekennzeichnet, daß die Anlenkpunkte (16) im Bereich der Innenkante der Halbsohlen (10,11) liegen.

5. Orthopädische Redressionsschiene nach einem der Ansprüche 1 bis 2, dadurch gekennzeichnet, daß die Feder eine um den Drehbolzen angeordnete Spiralfeder (18) ist, deren endseitige Schenkel (18a,18b) im wesentlichen parallel zur Außenkante der Halbsohlen (10,11) liegen.

6. Orthopädische Redressionsschiene nach einem der Ansprüche 1 bis 5, dadurch gekennzeichnet, daß die Halbsohlen (10,11) jeweils einen Winkel (14,15) aus biegefestem Material aufweisen, die jeweils endseitig von dem Drehbolzen (13) schwenkbar gehalten werden und deren freies Ende in Richtung der Innenkante und bis an diese heranreichend liegt, wobei diese Winkel (14,15) jeweils vorbereitete Anlenkpunkte (16) für die Druckfeder (17,18) aufweisen.

7. Orthopädische Redressionsschiene nach einem der Ansprüche 1 bis 6, dadurch gekennzeichnet, daß diese als Schuh- oder Stiefelteil ausgebildet ist.

## Claims

1. Orthopaedic correction splint for correcting the adduction of the forefoot or metatarsus with a half sole enclosing at least laterally the deformed part of the foot and a rear half sole (10,11), which are articulatedly interconnected so as to be swivellable about a vertical relative to the half sole plane defined by a fulcrum pin (13) and which possess a spring (17,18), whose spring force acts in the lateral direction upon the front half sole (10),
characterized in that
the fulcrum pin (13) is disposed on the external edge of the two half soles (10,11) and in that the spring is a compression spring (17,18), with the aid of which the half soles (10,11) are compressively loaded against each other.

2. Orthopaedic correction splint according to Claim 1,
characterized in that
the two half soles (10,11) each possess on their outside an extension, in which case the extensions overlap each other and are penetrated by the fulcrum pin (13).

3. Orthopaedic correction splint according to either Claim 1 or 2,
characterized in that,
at a distance from the fulcrum pin (13) towards the internal edge, each half sole (10,11) possesses at least one prepared coupling point (16) for the compression spring (17,18).

4. Orthopaedic correction splint according to Claim 3,
characterized in that
the coupling points (16) are located in the region of the internal edge of the half soles (10,11).

5. Orthopaedic correction splint according to either Claim 1 or 2,
characterized in that
the spring is a helical spring (18) arranged so as to surround the fulcrum pin, whose terminal legs (18a, 18b) are substantially parallel to the external edge of the half soles (10,11).

6. Orthopaedic correction splint according to any of Claims 1 to 5,
characterized in that
the half soles (10,11) are each provided with an angle plate (14,15) of a flexurally resistant material, which are in each case, at their ends, swivellably retained by the fulcrum pin (13) and whose free end is located in the direction of the internal edge and reaches as far as the latter, in which case these angle plates (14,15) each possess prepared coupling points (16) for the compressive spring (17,18).

7. Orthopaedic correction splint according to any of Claims 1 to 6,
characterized in that
the same is constructed in the form of a show or boot portion.

## Revendications

1. Atelle orthopédique de redressement pour la correction de l'adduction de la partie antérieure et de la partie centrale du pied avec une moitié de semelle antérieure qui entoure la partie déformée du pied au moins latéralement et une moitié de semelle postérieure (10, 11), moitiés de semelle qui sont reliées l'une à l'autre de manière articulée en pivotant autour d'une verticale par rapport au plan de la moitié de semelle qui est définie par un pivot (13) et qui présentent un ressort (17, 18) dont la force dans le sens latéral agit sur la moitié de semelle antérieure (10), **caractérisée en ce** que le pivot (13) est placé sur l'arête extérieure des deux moitiés de semelle (10, 11) et que le ressort est un ressort de pression (17, 18) par lequel les moitiés de semelle (10, 11) sont chargées en pression l'une contre l'autre sur le côté médian.

2. Atelle orthopédique de redressement selon la revendication 1, **caractérisée en ce** que les deux moitiés de semelle (10, 11) possédent un prolongement respectivement sur leur face extérieure, les prolongements se chevauchant et étant traversés par le pivot (13).

3. Atelle orthopédique de redressement selon l'une des revendications 1 à 2, **caractérisée en ce** que chacune des deux moitiés de semelle (10, 11) présente, à un certain écart du pivot (13) en direction de l'arête intérieure, au moins un point d'articulation préparé (16) pour le ressort de pression (17, 18).

4. Atelle orthopédique de redressement selon la revendication 3, **caractérisée en ce** que les points d'articulation (16) se situent dans la zone de l'arête intérieure des moitiés de semelle (10, 11).

5. Atelle orthopédique de redressement selon l'une des revendications 1 à 2, **caractérisée en ce** que le ressort est un ressort spiral (18) placé autour du pivot, ressort spiral dont les branches situées en extrémité (18a, 18b) sont substantiellement parallèles à l'arête extérieure des moitiés de semelle (10, 11).

6. Atelle orthopédique de redressement selon l'une des revendications 1 à 5, **caractérisée en ce** que les moitiés de semelle (10, 11) présentent respectivement un angle (14, 15) en matière résistante à la flexion, ces angles étant maintenus pivotants par le pivot (13) respecitvement en extrémité et dont l'extrémité libre est située dans le sens de l'arête intérieure et va jusqu'à celle-ci, ces angles (14, 15) présentant respectivement des points d'articulation préparés (16) pour le ressort de pression (17, 18).

7. Atelle orthopédique de redressement selon l'une des revendications 1 à 6, **caractérisée en ce** que celle-ci est configurée comme une partie de chaussure ou de botte.
